# FASCICULE DE BREVET EUROPEEN

(11) **EP 2 203 128 B1**
(45) Date de publication et mention de la délivrance du brevet: **16.01.2013**
(21) Numéro de dépôt: 08844179.5
(22) Date de dépôt: 24.10.2008
(51) Int. Cl.: A61F 2/26, A61F 2/00

(54) **IMPLANT CHIRURGICAL ET EN PARTICULIER SPHINCTER ARTIFICIEL A PRESSION REGULEE**
CHIRURGISCHES IMPLANTAT, INSBESONDERE KÜNSTLICHER SCHLIESSMUSKEL MIT ANGEPASSTEM DRUCK
SURGICAL IMPLANT, IN PARTICULAR ARTIFICIAL SPHINCTER WITH ADJUSTED PRESSURE

(30) Priorité: 24.10.2007 FR 0758552
(43) Date de publication de la demande: 07.07.2010
(73) Titulaire: Zephyr Surgical Implants, 69100 Villeurbanne (FR)
(72) Inventeur: GOMEZ-LLORENS, Christophe, 51370 Saint Brice Courcelles (FR)
(74) Mandataire: Thibault, Jean-Marc
(86) Numéro de dépôt international: PCT/FR2008/051927
(87) Numéro de publication internationale: WO 2009/056750

(56) Documents cités:
- GB-A- 2 016 117
- US-A- 4 197 835
- US-A- 4 994 020
- US-A1- 2002 022 759

## Description

La présente invention concerne le domaine technique des implants chirurgicaux adaptés pour contrôler le gonflement d'un élément gonflable en réponse à une pression d'un fluide.

La présente invention trouve une application particulièrement avantageuse pour constituer un sphincter urinaire artificiel pour l'homme ou la femme, un sphincter anal artificiel, un sphincter oesophagien ou pylorique artificiel ou constituer un anneau gastrique.

Dans l'application préférée d'un sphincter urinaire artificiel, il est connu divers systèmes pour apporter une solution à l'incontinence urinaire, en particulier masculine. Par exemple, le brevet US 4 222 377 décrit un sphincter urinaire artificiel comportant, en tant qu'élément gonflable, une manchette occlusive destinée à être implantée autour d'un segment du canal urétral. Cette manchette est constituée d'une partie externe renforcée, flexible, inextensible, sur laquelle adhère un coussin gonflable interne par un fluide sous pression.

Le sphincter urinaire artificiel comporte également un ballonnet régulateur de pression, destiné à être implanté dans l'espace pré-vésical, permettant à la manchette occlusive en position fermée, d'exercer autour du segment urétral des pressions proches des valeurs physiologiques. Cette compression des tissus de l'urètre doit empêcher le passage de l'urine et permettre le passage du flux sanguin, limitant ainsi les risques d'hypoxie tissulaire et de nécrose.

Le sphincter urinaire artificiel comporte également une pompe intrascrotale (peau des testicules) chez l'homme ou dans une des grandes lèvres chez la femme. La pompe comporte en partie inférieure, une poire que le patient comprime pour ouvrir le sphincter artificiel au moment de la miction. La pompe rassemble deux circuits en parallèle. Ainsi, cette pompe est équipée d'un premier cathéter reliant la pompe au ballonnet par l'intermédiaire d'un système antiretour à billes monté sur ressort. Cette pompe comporte un deuxième cathéter monté entre cette dernière et la manchette gonflable, via un système antiretour à billes. Par ailleurs, la manchette est reliée au ballonnet à travers un orifice calibré.

Pour assurer la continence du patient, du fluide circule librement entre la manchette et le ballonnet qui maintient une pression déterminée dans la manchette. Cette pression de la manchette sur l'urètre est suffisante pour assurer la continence, mais reste insuffisante pour arrêter le flux sanguin artériel et veineux. Les tissus comprimés restent irrigués, diminuant ainsi le risque d'ischémie.

Pour permettre au patient d'uriner, le fluide contenu dans la manchette doit être vidé dans le ballonnet afin de supprimer la compression de l'urètre. Pour ce faire, le patient actionne la poire de la pompe située dans le scrotum, de sorte que sous l'action de la pression de la poire, le conduit communiquant avec la manchette est fermé par le clapet à billes, et le liquide de la poire repousse le clapet à billes qui fermait le conduit communicant avec le ballonnet, permettant ainsi de remplir le ballonnet. Lors de la dépression de la poire, le conduit communiquant avec la manchette est libéré par aspiration de la bille, ce qui entraîne le vidage de la manchette dans la poire. Le conduit communiquant avec le ballonnet est bouché sous l'action du ressort. La pression sur la poire est répétée jusqu'à ce que la manchette soit vide. Le patient sait que la manchette est vide quand la poire de la pompe est plate, lui permettant ainsi d'uriner. Simultanément, le ballonnet remplit progressivement la manchette de la poire par l'orifice calibré. Le remplissage se fait lentement (à peu près 180 secondes) permettant au patient de vider complètement sa vessie avant que la manchette comprime de nouveau l'urètre.

Cet implant urinaire artificiel pose un certain nombre d'inconvénients.

En effet, l'implantation d'un tel implant urinaire artificiel nécessite de réaliser trois gestes chirurgicaux :
- une incision périnéale pour passer la manchette autour de l'urètre,
- une incision inguinale pour pénétrer dans le pelvis et placer le ballon régulateur de pression entre la vessie et les vaisseaux iliaques,
- une logette scrotale pour placer la pompe.

Les risques pour le patient sont :
- l'incision inguinale qui peut être à l'origine d'une infection nosocomiale qui imposera l'ablation de l'implant,
- la position du ballon régulateur de pression au contact de la veine iliaque qui peut être responsable d'une phlébite puis d'une embolie pulmonaire,
- le traitement anticoagulant prescrit pendant les 7 jours post-opératoires pour prévenir les phlébites qui peuvent être responsable d'une thrombopénie,
- l'hospitalisation d'une durée de 4 à 5 jours.

Par ailleurs, le ballon régulateur de pression est réalisé en un matériau élastique mais poreux, ce qui conduit à une perte irrémédiable du liquide de remplissage, ne permettant pas d'assurer une pression déterminée choisie pour la manchette.

En pratique, le sphincter urinaire artificiel tel que décrit ci-dessus est livré en kit à préparer par l'équipe chirurgicale au moment de l'implantation.

Le kit comprend notamment plusieurs ballons de différentes pressions, une pompe de commande, plusieurs manchettes de différentes tailles pour s'adapter à chaque type d'urètre, et une boite de connectique.

Avant l'implantation dans l'organisme, il convient de mesurer l'urètre et de choisir une taille pour la manchette, ainsi qu'un ballon de pression parmi ceux disponibles. La manchette, le ballon de pression et la pompe de commande doivent être remplis par un mélange précis d'eau stérile et de liquide de contraste. Ce mélange est à préparer par le chirurgien pour chaque intervention. Chaque élément doit être ensuite débullé. Par ailleurs, deux pinces que le chirurgien aura préalablement gainées seront placées sur chaque cathéter pour assurer l'étanchéité de chaque partie constituant ce kit. Cette conception impose d'aider le chirurgien par un infirmier pour chaque implantation d'un sphincter urinaire artificiel. En résumé, l'implantation d'un sphincter urinaire artificiel est une opération à risque pour le patient, relativement complexe et longue, et par conséquent coûteuse.

Le brevet US 4 994 020 qui reprend le principe de fonctionnement du brevet US 4 222 377 décrit un implant chirurgical comportant un élément gonflable, en réponse à une pression d'un fluide, un réservoir pour le fluide sous pression en communication avec l'élément gonflable pour réguler la pression dudit élément, une pompe commandée manuellement en communication d'une part avec l'élément gonflable via un obturateur unidirectionnel et, d'autre part, avec le réservoir via un obturateur unidirectionnel. Il est à noter qu'un réservoir additionnel est prévu pour participer à la régulation de la pression. Cet implant chirurgical comporte également un boîtier de commande intégrant le réservoir et la pompe manuelle. Ce boîtier de commande présente une ouverture de passage pour le fluide, en communication d'une part du côté intérieur du boîtier de commande, avec le réservoir et la pompe manuelle et, d'autre part, du côté extérieur du boîtier de commande, avec l'élément gonflable, à l'aide d'un cathéter monté entre l'élément gonflable et le boîtier de commande.

Le réservoir comporte une membrane mince extensible qui se distend sous la pression du fluide. Cette membrane mince extensible est typiquement réalisée en silicone, matériau dont la porosité n'est pas nulle et augmente avec le temps. De ce fait, il y a transfert vers le corps du patient de fluide hydraulique, de microparticules voire de bactéries accidentellement inoculées lors du remplissage de l'implant. En outre, ce transfert de fluide hydraulique entraîne à la longue une diminution de la pression dans la manchette, qui sera responsable de fuites urinaires chez le patient.

Il apparaît également que compte tenu de la conception de cet implant, il s'avère en pratique nécessaire d'adjoindre un réservoir additionnel pour disposer d'une pression de régulation suffisante au cours du temps. Ce réservoir additionnel réalisé à l'aide d'une membrane extensible présente les mêmes inconvénients que le réservoir principal tout en rendant l'implant volumineux pour être implanté dans le scrotum.

La présente invention vise à remédier aux inconvénients de l'état technique, en proposant un nouvel implant chirurgical, conçu pour limiter les risques liés à la mise en place d'un tel implant chirurgical, tout en étant adapté pour assurer une régulation précise, stable et constante dans le temps de la pression d'un élément gonflable.

Un autre objet de l'invention est de proposer un nouvel implant chirurgical qui minimise voire supprime les pertes du fluide de régulation.

Un autre objet de l'invention est de proposer un nouvel implant chirurgical pratiquement prêt à l'utilisation avant chaque implantation.

Un autre objet de l'invention est de proposer un nouvel implant chirurgical conçu pour présenter un fonctionnement sûr et fiable, et notamment une pression de régulation stable dans le temps.

Pour atteindre de tels objectifs, l'objet de l'invention concerne un implant chirurgical comportant :
- un élément gonflable, en réponse à une pression d'un fluide,
- un réservoir pour le fluide sous pression en communication avec l'élément gonflable pour réguler la pression dudit élément,
- une pompe commandée manuellement en communication d'une part avec l'élément gonflable via un obturateur unidirectionnel et, d'autre part, avec le réservoir via un obturateur unidirectionnel,
- un boîtier de commande intégrant le réservoir et la pompe manuelle, le boîtier de commande présentant une ouverture de passage pour le fluide, en communication d'une part du côté intérieur du boîtier de commande, avec le réservoir et la pompe manuelle et, d'autre part, du côté extérieur du boîtier de commande, avec uniquement l'élément gonflable, à l'aide d'un cathéter monté entre l'élément gonflable et le boîtier de commande.

Selon l'invention, le réservoir est formé par une chambre étanche de fluide sous pression, délimitée par un piston mobile sollicité élastiquement par un ressort.

Selon une caractéristique préférée de réalisation, le réservoir est formé par une chambre étanche de fluide sous pression, délimitée par un piston mobile sollicité élastiquement.

Par exemple, le réservoir comporte un cylindre dans lequel est montée une membrane d'étanchéité fixée entre le piston et le cylindre.

Selon un exemple préféré de réalisation, le piston est sollicité élastiquement par un ressort monté à l'intérieur du cylindre en appui sur un support de montage.

Il est à noter que le ressort baigne dans un milieu fluide confiné différent du fluide sous pression et formant un volume de compensation par rapport au volume de la chambre.

Par exemple, le piston délimite du côté opposé de la chambre, un volume de compensation fermé par une paroi déformable confinant le milieu fluide dans lequel baigne le ressort.

Selon une autre caractéristique de l'objet de l'invention, le cylindre est fermé à l'opposé du piston par un fond délimitant :
- un circuit de communication entre la chambre et l'ouverture de passage,
- un circuit de communication entre la chambre et la pompe,
- et un circuit de communication entre la pompe et l'ouverture de passage.

Avantageusement, la pompe comporte une paroi d'actionnement mobile montée sur le cylindre et délimitant intérieurement une réserve de fluide à volume variable, en communication avec les circuits de communication et entre la pompe et l'ouverture de passage, et entre la pompe et la chambre du réservoir.

Pour des raisons de compacité, la paroi de la pompe s'étend sensiblement sur toute la hauteur du cylindre en étant pourvu de stries d'aide à son actionnement.

Selon une autre caractéristique de l'invention :
- le circuit de communication entre la chambre et l'ouverture de passage est pourvu d'un orifice calibré de fuite pour réguler la pression de l'élément gonflable,
- le circuit de communication entre la pompe et la chambre est pourvu de l'obturateur unidirectionnel rappelé élastiquement à sa position fermée de repos.

Pour permettre l'activation et la désactivation de l'implant, le boîtier de commande comporte un interrupteur de commande de fermeture du circuit de communication entre le réservoir et l'élément gonflable.

De préférence, l'interrupteur de commande est accessible de part et d'autre du boîtier de commande.

Selon un exemple d'application pour un sphincter urinaire artificiel, l'élément gonflable se présente sous la forme d'une manchette apte à entourer un organe pourvu d'un passage interne, de manière à être fermé ou ouvert par constriction par ledit élément gonflable.

Diverses autres caractéristiques ressortent de la description faite ci-dessous en référence aux dessins annexés qui montrent, à titre d'exemples non limitatifs, des formes de réalisation de l'objet de l'invention.
La Figure **1** est une vue en coupe élévation montrant un exemple de réalisation d'un implant chirurgical conforme à l'invention.
La Figure **2** est un schéma illustrant le principe de fonctionnement de l'implant chirurgical conforme à l'invention.
La Figure **3** est une vue en coupe élévation d'un implant chirurgical dans une position caractéristique d'activation.
Les Figures **4** et **5** sont des vues en coupe transversale prises sensiblement selon les lignes respectivement IV et V de la Figure **7****.**
La Figure **6** est une vue en élévation prise selon un autre plan de coupe de l'implant chirurgical conforme à l'invention.
La Figure **7** est une vue en coupe élévation de l'implant chirurgical montrant le réservoir vide.

Tel que cela ressort plus précisément des Figures, l'objet de l'invention concerne un implant chirurgical **1** comportant un élément gonflable **2** en réponse à une pression d'un fluide. Dans l'exemple de réalisation illustré, l'élément gonflable **2** se présente sous la forme d'une manchette **3** apte à entourer un organe pourvu d'un passage interne destiné à être fermé ou ouvert par constriction par ledit élément gonflable **2.** Dans cette application, l'implant chirurgical est apte à former un sphincter artificiel, en particulier urinaire.

Selon cet exemple de réalisation, la manchette **3** comporte une bande flexible inextensible sur laquelle adhère un coussin **4** gonflable par un fluide sous pression. La manchette **3** est apte à être repliée sur elle-même et maintenue en position refermée pour entourer un organe pourvu d'un passage interne devant être fermé ou ouvert à volonté.

L'élément gonflable **2** comporte une tubulure **5** de raccordement entre le coussin gonflable **4** et un cathéter ou un conduit **6** destiné à être relié à un boîtier de commande **7.**

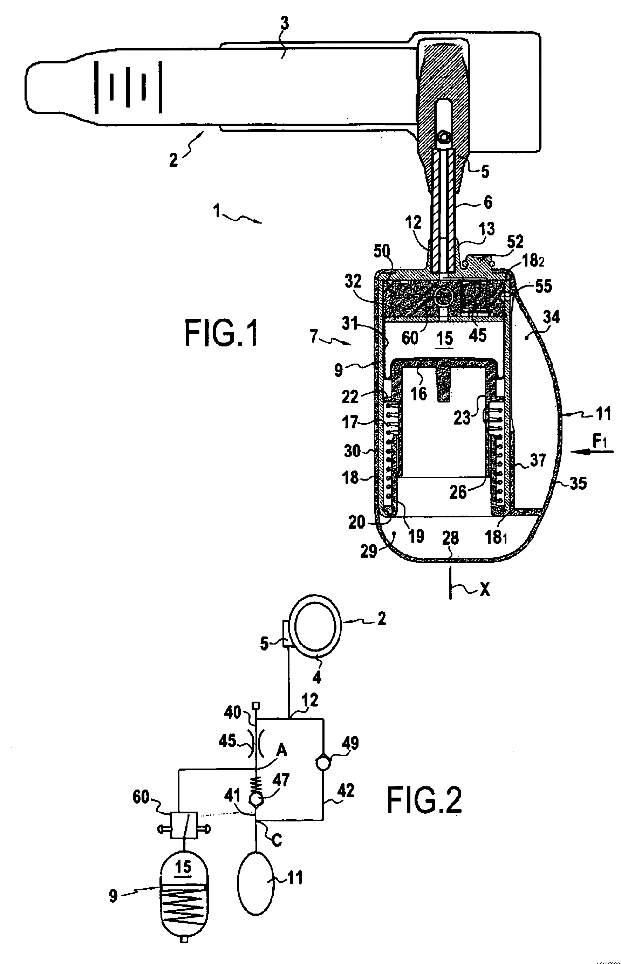

Selon une caractéristique avantageuse de l'invention, le boîtier de commande **7** intègre un réservoir **9** pour le fluide sous pression et une pompe **11** commandée manuellement pour assurer la mise en circulation du fluide comme cela sera expliqué dans la suite de la description. Le boîtier de commande **7** comporte une ouverture de passage **12** pour le fluide sur laquelle est raccordé le cathéter **6.** A cet effet, le boîtier de commande **7** comporte une tubulure **13** de raccordement pour le cathéter **6,** délimitant intérieurement l'ouverture de passage **12.** Tel que cela ressort plus précisément de la Figure **1****,** l'ouverture de passage **12** se trouve en communication d'une part du côté extérieur du boîtier de commande **7** avec uniquement l'élément gonflable **2** par l'intermédiaire du cathéter **6,** et d'autre part, du côté intérieur du boîtier de commande **7,** avec le réservoir **9** et la pompe manuelle **11** par des circuits de circulation du fluide qui seront décrits plus précisément dans la suite de la description.

Selon une caractéristique préférée de réalisation, le réservoir **9** est formé par une chambre étanche **15** contenant le fluide sous pression, présentant un volume variable sous l'action d'un piston mobile **16** sollicité élastiquement. Le piston mobile **16** est sollicité élastiquement par l'intermédiaire d'un ressort **17** par exemple à spirale. Dans l'exemple illustré, le réservoir **7** comporte un cylindre **18** à paroi fixe ou rigide, par exemple de section droite transversale circulaire présentant un axe de symétrie longitudinale **X.** Le piston mobile **16** est monté coulissant à l'intérieur du cylindre **18** en étant sollicité par le ressort **17** monté en appui sur un support **19** fixé sur le cylindre **18.**

Dans l'exemple illustré, le support **19** se présente sous la forme d'une bague pourvue à sa base d'un rebord saillant **20** fixé sur une extrémité par exemple **18₁** du cylindre **18.** Le support **19** s'étend ainsi à l'intérieur du cylindre **18** sur une partie de sa longueur, en délimitant avec ce dernier un logement pour le ressort **17.** Le ressort **17** se trouve ainsi en appui entre le rebord saillant **20** et une collerette saillante **22** s'étendant à l'extérieur d'une jupe **23** prolongeant à l'équerre la paroi de piston **16.** La jupe **23** est montée pour s'étendre à l'intérieur du support **19** afin de servir de guidage au piston **16** par l'intermédiaire d'un rebord épaulé **26** porté par l'extrémité libre de la jupe **23.** Le ressort **17** est calibré pour maintenir le fluide à une pression déterminée de préférence supérieure à la pression régnant dans le passage interne mais inférieure à la pression sanguine de manière à fermer le passage interne de l'organe tout en permettant la circulation sanguine. Typiquement, le ressort **17** est calibré pour maintenir le fluide à une pression déterminée comprise entre 61 et 70 mbar.

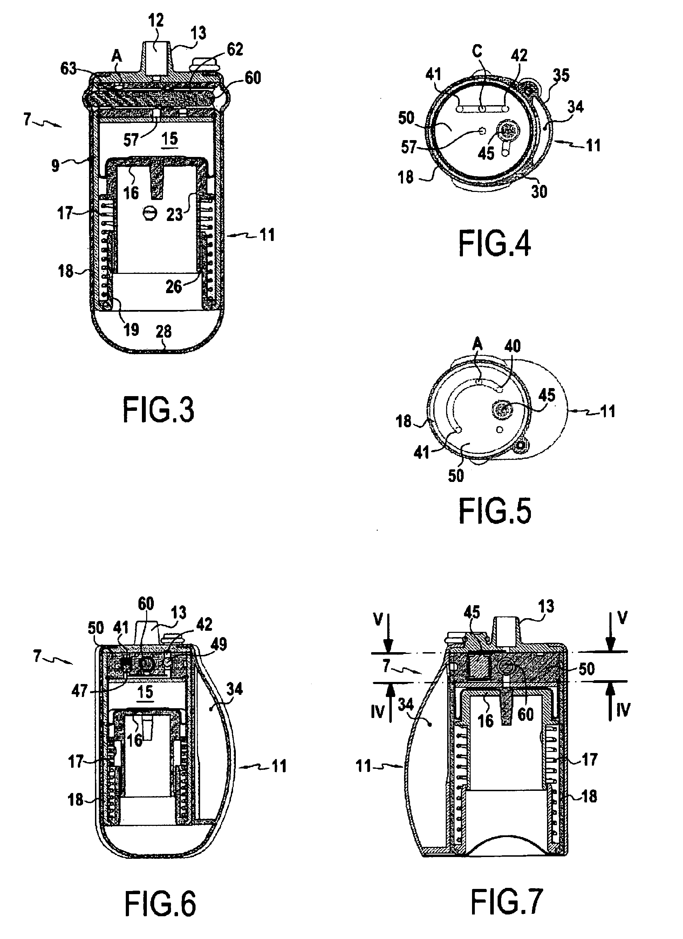

Selon une caractéristique préférée de réalisation, le ressort **17** baigne dans un milieu fluide tel que du sérum physiologique par exemple. Ce milieu fluide qui est différent du fluide sous pression contenu dans le réservoir **9** se trouve confiné par une membrane externe ou une peau **28** venant fermer ainsi l'extrémité **18₁** du cylindre. Ce milieu fluide est destiné à former un volume de compensation **29** par rapport au volume de la chambre **15.** Tel que cela ressortira de la description qui suit, cette membrane externe **28** est destinée à évoluer entre des positions extrêmes concave (Figure **7** montrant la chambre **15** vide) ou convexe (Figure **1**) en fonction de la position du piston **16.** De préférence, cette membrane externe **28** s'étend dans le prolongement d'un manchon tubulaire **30** entourant le cylindre **18.** Le manchon tubulaire **30** se trouve fermé à une extrémité par la membrane externe **28.**

Tel que cela ressort plus précisément des Figures **1** et **7****,** le piston **16** est monté de manière étanche à l'intérieur du cylindre à l'aide de préférence d'une membrane interne ou d'une peau tubulaire **31** fixée entre le piston **16** et le cylindre **18.** Cette membrane interne **31** est fixée sur le piston **16** en étant plaquée dans la paroi interne du cylindre **19** jusqu'à un fond **32** de fermeture du cylindre **18.** Par exemple, l'une des extrémités de la membrane interne **31** est fixée sur le piston **16** tandis que l'autre extrémité est coincée entre le fond **32** et le cylindre **18.**

Cette membrane interne **31** roule ainsi entre le piston **16** et le cylindre **18.** Il est à noter que le piston **16** présente un diamètre inférieur par rapport au diamètre du cylindre **18,** de manière que le piston **16** délimite à sa périphérie avec le cylindre **18,** un logement pour la membrane interne **31.** La chambre **15** du réservoir **9** est donc délimitée à l'intérieur du cylindre rigide **18** entre le piston mobile **16** et le fond de fermeture **32** du cylindre, ou compte tenu du montage de la membrane interne **31,** par le volume interne délimité par cette membrane interne **31** en combinaison avec le fond de fermeture **32.**

Selon une caractéristique avantageuse de réalisation, la pompe manuelle **11** forme une chambre **34** de confinement du fluide sous pression, accessible de l'extérieur de l'organe de commande **7.**

Avantageusement la pompe manuelle **11** comporte une paroi d'actionnement mobile **35** montée à l'extérieur du cylindre **18** sur une plage angulaire limitée inférieure à 180° par exemple, comme cela ressort clairement des Figures **4** et **5****.** La paroi d'actionnement **35** s'étend à distance du cylindre **18** pour délimiter la réserve **34** de fluide sous pression à volume variable. La paroi d'actionnement **35** est pourvue extérieurement de protubérances ou de stries facilitant la localisation pour le patient. L'actionnement de la pompe **11** est donc assuré par un appui sur la paroi déformable **35** selon la flèche **F₁** (**Fig. 1**) de sorte que l'action sur la commande de la pompe est sensiblement perpendiculaire à l'axe longitudinal **X** de l'organe de commande **7.**

Avantageusement, la paroi déformable **35** s'étend à distance d'une partie du manchon **30** entourant le cylindre **18.** En d'autres termes, la réserve de fluide **34** de cette pompe manuelle forme une excroissance sur un côté du cylindre **18,** par exemple sur toute sa hauteur. De préférence, le manchon **30,** la paroi d'actionnement **35** et la membrane externe **28** forment une unique pièce montée sur le cylindre **18** et réalisée par exemple par une pièce en silicone moulée. Bien entendu, la réserve de fluide sous pression **34** est isolée par rapport au milieu fluide du volume de compensation **29.**

Le boîtier de commande **7** comporte avantageusement :
- un circuit **40** de communication entre la chambre **15** du réservoir **9** et l'ouverture de passage **12,**
- un circuit de communication **41** entre la chambre **15** du réservoir **9** et la pompe **11** et plus précisément la réserve de fluide **34,**
- et un circuit de communication **42** entre la pompe **11** et plus précisément la réserve de fluide **34** et l'ouverture de passage **12.**

Il doit être compris que l'implant chirurgical **1** présente ainsi un circuit fermé de circulation pour un fluide sous pression composé du coussin gonflable **4,** du cathéter **6,** de la chambre **15** du réservoir **9,** de la réserve de fluide **34** de la pompe **11** et des circuits de communication **40** à **42** qui vont être décrits plus précisément dans la suite de la description. Par exemple, le fluide sous pression circulant dans tel circuit peut être du sérum physiologique.

Tel que cela ressort plus précisément de la **Figure 2****,** le circuit **40** de communication entre la chambre **15** et l'ouverture de passage **12** est pourvu d'un orifice calibré de fuite **45** adapté pour réguler la pression de l'élément gonflable **2** comme cela sera expliqué dans la suite de la description. Le circuit de communication **41** entre la réserve de fluide **34** de la pompe **11** et la chambre **15** est pourvu d'un obturateur unidirectionnel **47** rappelé élastiquement à sa position fermée de repos. Par exemple, l'obturateur unidirectionnel **47** est une soupape en appui sur son siège par l'intermédiaire d'un ressort. L'obturateur **47** est fermé lorsque la pression du côté de la pompe **11** (point **C**) est inférieure à la pression au point **A** situé du côté de la chambre **15.** A contrario, l'obturateur unidirectionnel **47** est en position d'ouverture lorsque la pression au point **C** est supérieure à la pression au point **A.**

Le circuit de communication **42** entre la réserve de fluide **34** de la pompe **11** et l'ouverture de passage **12** comporte un obturateur unidirectionnel **49** tel qu'un clapet de vidange comme cela sera expliqué dans la suite de la description. Le clapet de vidange **49** est en position fermée lorsque la pression au point **C** est supérieure à la pression au niveau de l'ouverture de passage **12.** A l'inverse, le clapet **49** est en position d'ouverture lorsque la pression au niveau de l'ouverture de passage **12** est supérieure à la pression au point **C.**

Selon une autre caractéristique avantageuse de l'objet de l'invention, les circuits de communication **40, 41** et **42** sont aménagés dans un bloc de distribution **50** associé au fond du cylindre **18.** De manière plus précise, ce bloc de distribution **50** est monté entre la paroi de fond **32** délimitant directement la chambre **15** et un couvercle **52** venant fermer l'extrémité **18₂** opposée de l'extrémité **18₁** du cylindre. Le couvercle **52** est pourvu avantageusement de la tubulure de raccordement **13** délimitant l'ouverture de passage **12.** De préférence, le manchon tubulaire **30** est fixé par son bord libre sur le couvercle **52.**

Le bloc de distribution **50** comporte des conduits ou des canaux aménagés pour réaliser les circuits **40** à **42** tels que décrits ci-dessus. Ce bloc de distribution **50** intègre également les obturateurs **45, 47 et 49.** Tel que cela ressort plus précisément de la Figure **1****,** un trou **55** est aménagé dans la paroi du cylindre **18** de manière à assurer une communication au point C entre la réserve **34** de la pompe manuelle **11** et les circuits **41** et **42 (****Fig. 4****).** De même, la paroi de fond 32 est pourvue d'un trou de passage **57** débouchant dans le bloc de distribution **50** de manière que la chambre **15** du réservoir se trouve reliée au point **A** de communication entre les circuits **40** et **41 (****Fig. 3** et **5****).**

Selon une caractéristique avantageuse de réalisation, un interrupteur **60** est placé pour permettre d'isoler le réservoir **9** par rapport à l'élément gonflable **2** de manière à placer le boîtier de commande **7** dans un état de désactivation. Tel que cela ressort plus précisément à la Figure **3****,** l'interrupteur **60** se présente sous la forme d'un axe de commande placé à l'intérieur d'un alésage **62** apte à assurer une communication entre le trou de sortie **57** de la chambre **15** et le point **A** à savoir notamment le point de raccordement du circuit **40** de communication avec l'ouverture de passage **12,** via l'orifice calibré **45.** L'alésage **62** est aménagé dans le bloc de distribution **50** selon une direction perpendiculaire à l'axe **X** et de préférence perpendiculaire également à la direction d'appui **F₁** sur le réservoir **11 (****Fig. 1****).** De préférence, l'alésage **62** débouche de part et d'autre du bloc de distribution **50** de manière que l'axe **60** puisse être accessible de part et d'autre du boîtier de commande **7,** à travers le manchon **30.** L'axe **60** est pourvu de joints d'étanchéité **63** adaptés de manière que pour des positions d'activation et de désactivation, l'axe **60** occupe des positions assurant respectivement la communication et la non communication entre la chambre **15** et le point **A** et par suite, l'élément gonflable **2.** Le déplacement de l'axe **60** selon un sens permet de placer le boîtier de commande **7** dans sa position désactivée (obturation entre le point **A** et le trou de sortie **57)** tandis que son déplacement dans un sens contraire permet de placer le boîtier de commande **7** dans sa position activée (communication entre le point **A** et le trou de sortie **57,** **Fig. 3****).** Bien entendu, l'axe **60** coopère avec des butées pour occuper des positions stables activée et désactivée.

Le fonctionnement de l'implant chirurgical **1** découle directement de la description qui précède.

La description qui suit est décrite pour un sphincter urinaire artificiel. Dans ce cas, la manchette est implantée autour d'un segment du canal urétral, tandis que le boîtier de commande **7** est implanté intra-scrotale.

Il doit être considéré que le patient est continent lorsque la manchette **2** est gonflée et occlusive. Pour cette position, le fluide circule librement entre le réservoir **9** et plus précisément la chambre **15** et le coussin **4** de la manchette gonflable **2.** Il est à noter que le ressort **17** est dimensionné de manière à maintenir une pression comprise entre 61 et 70 mbar dans l'élément gonflable **2.** Cette pression de la manchette sur l'urètre est suffisante pour assurer la continence mais reste insuffisante pour arrêter le flux sanguin artériel et veineux. Les tissus comprimés restent irrigués.

Lorsque le patient veut uriner, il convient de vider l'élément gonflable **2** dans le réservoir **9** pour supprimer la compression de l'urètre. A cet effet, le patient actionne la pompe **11** en assurant un appui sur la paroi déformable **35.**

Sous l'action de la pression de la pompe **11,** le clapet de vidange **49** est fermé et le fluide contenu dans la réserve **34** de la pompe **11** repousse la soupape **47** qui fermait le circuit **41** permettant ainsi de remplir la chambre **15** du réservoir **9.**

A la dépression de la pompe **11 :**
- le circuit **42** s'ouvre dans la mesure où le clapet de vidange **49** occupe sa position d'ouverture puisque la pression du fluide à l'intérieur du coussin gonflable **4** est supérieure à la pression à l'intérieur de la réserve **34** de la pompe **11.** L'élément gonflable **2** se vide dans la réserve **34** de la pompe **11.**
- le circuit **41** est fermé sous l'action du ressort de la soupape **47** puisque la pression à l'intérieur de la pompe **11** est inférieure à la pression de la chambre **15.**

La pression sur la pompe **11** est répétée jusqu'à ce que l'élément gonflable **2** soit vide. Le patient sait que l'élément gonflable **2** est vide lorsque la pompe est plate. Le patient peut alors uriner dans la mesure où le coussin gonflable **4** n'assure plus de pression. La pompe **11** permet ainsi de transférer le fluide provenant du coussin gonflable **4** dans la chambre **15** qui bien entendu est dimensionnée pour permettre le vidage complet du coussin gonflable **4.**

Concomitamment, le circuit **40** reste en permanence ouvert, de sorte que le réservoir **9** remplit progressivement, par sa chambre **15,** l'élément gonflable **2** mais également la réserve **34** de la pompe **11** par l'intermédiaire du circuit **42.** Le remplissage est réalisé lentement par l'intermédiaire de l'orifice calibré **45** (à peu près 180 secondes). Le patient a le temps de vider complètement sa vessie, avant que la manchette **3** comprime à nouveau l'urètre comme expliqué ci-dessus, la chambre **15** est dimensionnée pour permettre le remplissage du coussin gonflable **4** afin que la manchette occupe sa position occlusive. En pratique, la chambre **15** possède un volume de fluide bien supérieur au volume de fluide nécessaire pour remplir le coussin gonflable **4.**

Le sphincter urinaire artificiel peut être bloqué en plaçant l'interrupteur **60** en position désactivée. Cette désactivation est nécessaire pendant une durée de l'ordre de 2 mois après l'implantation pour éviter toute compression de l'urètre déjà traumatisé par le geste chirurgical.

Il ressort de la description qui précède que l'implant chirurgical **1** nécessite, pour son implantation, une seule incision périnéale pour placer l'élément gonflable **2** autour de l'urètre et faire glisser le boîtier de commande **7** dans une logette scrotale. Par rapport à la technique antérieure, il n'y a pas d'abord du pelvis, tous les risques liés à la mise en place d'un ballon régulateur de pression entre la vessie et les vaisseaux iliaques sont écartés et une hospitalisation courte est possible. Cette implantation facilitée est due à l'intégration dans le boîtier de commande **7** du réservoir **9** et de la pompe manuelle **11.** Par ailleurs, la mise en oeuvre d'une chambre étanche variable sous l'action d'un piston mobile élastiquement permet de disposer d'un système régulateur de pression particulièrement fiable et efficace. Par ailleurs, l'implant chirurgical **1** est pratiquement prêt à l'implantation, il suffit de le remplir de sérum physiologique avant son implantation.

Dans la description qui précède l'élément gonflable **2** se présente sous la forme d'une manchette **3** apte à entourer un organe pourvu d'un passage interne, de manière à constituer un sphincter artificiel. Bien entendu, il peut être envisagé que l'élément gonflable se présente sous la forme d'un élément allongé apte à former un implant pénien.

## Revendications

1. Implant chirurgical comportant :
- un élément gonflable **(2),** en réponse à une pression d'un fluide,
- un réservoir **(9)** pour le fluide sous pression en communication avec l'élément gonflable pour réguler la pression dudit élément,
- une pompe commandée manuellement **(11)** en communication d'une part avec l'élément gonflable via un obturateur unidirectionnel **(49)** et, d'autre part, avec le réservoir via un obturateur unidirectionnel **(47),**
- un boîtier de commande **(7)** intégrant le réservoir **(9)** et la pompe manuelle **(11),** le boîtier de commande présentant une ouverture de passage **(12)** pour le fluide, en communication d'une part du côté intérieur du boîtier de commande **(7),** avec le réservoir **(9)** et la pompe manuelle **(11)** et, d'autre part, du côté extérieur du boîtier de commande, avec uniquement l'élément gonflable **(2),** à l'aide d'un cathéter **(6)** monté entre l'élément gonflable **(2)** et le boîtier de commande **(7) caractérisé en ce que** le réservoir **(9)** est formé par une chambre étanche **(15)** de fluide sous pression, délimitée par un piston mobile **(16)** sollicité élastiquement par un ressort **(17).**

2. Implant chirurgical selon la revendication 1, **caractérisé en ce que** la chambre étanche **(15)** de fluide sous pression est délimitée à l'intérieur d'un cylindre rigide **(18)** entre un fond de fermeture **(32)** du cylindre et le piston mobile **(16).**

3. Implant chirurgical selon la revendication 2, **caractérisé en ce qu'** une membrane d'étanchéité **(31)** est fixée entre le piston **(16)** et le cylindre **(18).**

4. Implant chirurgical selon la revendication 2 ou 3, **caractérisé en ce que** le ressort **(17)** est monté à l'intérieur du cylindre **(18)** en appui sur un support de montage **(19).**

5. Implant chirurgical selon la revendication 4, **caractérisé en ce que** le ressort **(17)** baigne dans un milieu fluide confiné différent du fluide sous pression et formant un volume de compensation **(29)** par rapport au volume de la chambre **(15).**

6. Implant chirurgical selon la revendication 5, **caractérisé en ce que** le piston **(16)** délimite du côté opposé de la chambre **(15),** un volume de compensation **(29)** fermé par une paroi déformable **(28)** confinant le milieu fluide dans lequel baigne le ressort.

7. Implant chirurgical selon la revendication 3, **caractérisé en ce que** le cylindre **(18)** est fermé à l'opposé du piston **(16)** par un fond délimitant :
- un circuit **(40)** de communication entre la chambre **(15)** et l'ouverture de passage **(12),**
- un circuit de communication **(41)** entre la chambre **(15)** et la pompe **(11),**
- et un circuit de communication **(42)** entre la pompe **(11)** et l'ouverture de passage **(12).**

8. Implant chirurgical selon la revendication 1 ou 7, **caractérisé en ce que** la pompe **(11)** comporte une paroi d'actionnement mobile **(35)** montée sur le cylindre **(18)** et délimitant intérieurement une réserve de fluide à volume variable **(34),** en communication avec les circuits de communication **(42)** et **(41)** entre la pompe **(11)** et l'ouverture de passage **(12),** et entre la pompe **(11)** et la chambre (15) du réservoir **(9).**

9. Implant chirurgical selon la revendication 8, **caractérisé en ce que** la paroi **(35)** de la pompe s'étend sensiblement sur toute la hauteur du cylindre **(18)** en étant pourvu de stries d'aide à son actionnement.

10. Implant chirurgical selon la revendication 7 ou 8, **caractérisé en ce que** :
- le circuit de communication **(40)** entre la chambre **(15)** et l'ouverture de passage **(12)** est pourvu d'un orifice calibré de fuite **(45)** pour réguler la pression de l'élément gonflable **(2),**
- le circuit de communication **(41)** entre la pompe **(11)** et la chambre **(15)** est pourvu de l'obturateur **(47)** unidirectionnel rappelé élastiquement à sa position fermée de repos.

11. Implant chirurgical selon la revendication 1 ou 10, **caractérisé en ce qu'**il comporte un interrupteur de commande **(60)** de fermeture du circuit de communication **(40)** entre le réservoir **(9)** et l'élément gonflable **(2).**

12. Implant chirurgical selon la revendication 11, **caractérisé en ce que** l'interrupteur de commande **(60)** est accessible de part et d'autre du boîtier de commande **(7).**

13. Implant chirurgical selon l'une des revendications 1 à 12, **caractérisé en ce que** l'élément gonflable **(2)** se présente sous la forme d'une manchette **(3)** apte à entourer un organe pourvu d'un passage interne, de manière à être fermé ou ouvert par constriction par ledit élément gonflable.

## Claims

1. A surgical implant including:
- an inflatable member (2), in response to a pressure of a fluid,
- a reservoir (9) for the pressurized fluid in communication with the inflatable member for regulating the pressure of said member,
- a manually controlled pump (11) in communication with the inflatable member via a unidirectional obturator (49) on the one hand and with the reservoir via a unidirectional obturator (47) on the other hand,
- a control box (7) integrating the reservoir (9) and the manual pump (11), the control box having an opening (12) for letting through the fluid, in communication with the interior side of the control box (7), with the reservoir (9) and the manual pump (11) on the one hand and, on the exterior side of the control box, with only the inflatable member (2), on the other hand, by means of a catheter (6) mounted between the inflatable member (2) and the control box (7), **characterized in that** the reservoir (9) is formed by a leakproof pressurized fluid chamber (15), delimited by a mobile piston (16) elastically urged by a spring (17).

2. The surgical implant according to claim 1, **characterized in that** the leakproof pressurized fluid chamber (15) is delimited inside a rigid cylinder (18) between a closing bottom (32) of the cylinder and the mobile piston (16).

3. The surgical implant according to claim 2, **characterized in that** a seal membrane (31) is attached between the piston (16) and the cylinder (18).

4. The surgical implant according to claim 2 or 3, **characterized in that** the spring (17) is mounted inside the cylinder (18) bearing upon a mounting support (19).

5. The surgical implant according to claim 4, **characterized in that** the spring (17) is immersed in a confined fluid medium, different from the pressurized fluid, and forming a compensation volume (29) with respect to the volume of the chamber (15).

6. The surgical implant according to claim 5, **characterized in that** the piston (16) delimits on the opposite side of the chamber (15), a compensation volume (29) closed by a deformable wall (28) confining the fluid medium in which the spring is immersed.

7. The surgical implant according to claim 3, **characterized in that** the cylinder (18) is closed opposite to the piston (16) by a bottom delimiting:
- a circuit (40) for communicating between the chamber (15) and the passage opening (12),
- a circuit (41) for communicating between the chamber (15) and the pump (11),
- a circuit (42) for communicating between the pump (11) and the passage opening (12).

8. The surgical implant according to claim 1 or 7, **characterized in that** the pump (11) includes a mobile actuation wall (35) mounted on the cylinder (18) and interiorly delimiting a reserve (34) of fluid with a variable volume, in communication with the circuits (42) and (41) for communicating between the pump (11) and the passage opening (12), and between the pump (11) and the chamber (15) of the reservoir (9).

9. The surgical implant according to claim 8, **characterized in that** the wall (35) of the pump substantially extends over the whole height of the cylinder (18), while being provided with striations for assisting with its actuation.

10. The surgical implant according to claim 7 or 8, **characterized in that**:
- the circuit (40) for communicating between the chamber (15) and the passage opening (12) is provided with a calibrated leak orifice (45) for regulating the pressure of the inflatable element (2),
- the circuit (41) for communicating between the pump (11) and the chamber (15) is provided with the unidirectional obturator (47) elastically returned to its closed rest position.

11. The surgical implant according to claim 1 or 10, **characterized in that** it includes a control switch (60) for closing the circuit (40) for communicating between the reservoir (9) and the inflatable element (2).

12. The surgical implant according to claim 11, **characterized in that** the control switch (60) is accessible from either side of the control box (7).

13. The surgical implant according to one of claims 1 to 12, **characterized in that** the inflatable element (2) appears as a sleeve (3) capable of surrounding a member provided with an internal passage, so as to be closed or open by constriction by said inflatable element.

## Patentansprüche

1. Chirurgisches Implantat, umfassend:
- ein als Reaktion auf einen Druck eines Fluids aufblasbares Element (2),
- ein Reservoir (9) für das Druckfluid, das mit dem aufblasbaren Element in Verbindung steht, um den Druck des Elements zu regulieren,
- eine manuell betätigte Pumpe (11), die einerseits über einen Einweg-Verschluß (49) mit dem aufblasbaren Element und andererseits über einen Einweg-Verschluß (47) mit dem Reservoir in Verbindung steht,
- ein Steuergehäuse (7), das das Reservoir (9) und die manuelle Pumpe (11) beinhaltet, wobei das Steuergehäuse eine Durchgangsöffnung (12) für das Fluid aufweist, die einerseits, auf der Innenseite des Steuergehäuses (7), mit dem Reservoir (9) und der manuellen Pumpe (11) und andererseits, auf der Außenseite des Steuergehäuses, mit Hilfe eines Katheters (6), der zwischen dem aufblasbaren Element (2) und dem Steuergehäuse (7) angebracht ist, lediglich mit dem aufblasbaren Element (2) in Verbindung steht, **dadurch gekennzeichnet, daß** das Reservoir (9) von einer dichten Kammer (15) für Druckfluid gebildet ist, die durch einen durch eine Feder (17) elastisch beaufschlagten beweglichen Kolben (16) begrenzt ist.

2. Chirurgisches Implantat nach Anspruch 1, **dadurch gekennzeichnet, daß** die dichte Druckfluidkammer (15) innerhalb eines starren Zylinders (18) zwischen einem Boden zum Verschließen (32) des Zylinders und dem beweglichen Kolben (16) begrenzt ist.

3. Chirurgisches Implantat nach Anspruch 2, **dadurch gekennzeichnet, daß** eine Dichtungsmembran (31) zwischen dem Kolben (16) und dem Zylinder (18) befestigt ist.

4. Chirurgisches Implantat nach Anspruch 2 oder 3, **dadurch gekennzeichnet, daß** die Feder (17) innerhalb des Zylinders (18) in Anlage an einem Montagehalter (19) angebracht ist.

5. Chirurgisches Implantat nach Anspruch 4, **dadurch gekennzeichnet, daß** die Feder (17) in einem begrenzten flüssigen Medium badet, das sich von dem Druckfluid unterscheidet und das ein Ausgleichsvolumen (29) zu dem Volumen der Kammer (15) bildet.

6. Chirurgisches Implantat nach Anspruch 5, **dadurch gekennzeichnet, daß** der Kolben (16) auf der von der Kammer (15) abgewandten Seite ein Ausgleichsvolumen (29) begrenzt, das durch eine verformbare Wand (28), die das flüssige Medium, in dem die Feder badet, begrenzt, verschlossen ist.

7. Chirurgisches Implantat nach Anspruch 3, **dadurch gekennzeichnet, daß** der Zylinder (18) gegenüber dem Kolben (16) durch einen Boden verschlossen ist, der begrenzt:
- einen Verbindungskreis (40) zwischen der Kammer (15) und der Durchgangsöffnung (12),
- einen Verbindungskreis (41) zwischen der Kammer (15) und der Pumpe (11) und
- einen Verbindungskreis (42) zwischen der Pumpe (11) und der Durchgangsöffnung (12).

8. Chirurgisches Implantat nach Anspruch 1 oder 7, **dadurch gekennzeichnet, daß** die Pumpe (11) eine bewegliche Betätigungswand (35) umfaßt, die an dem Zylinder (18) angebracht ist und innen eine Fluidreserve mit veränderlichem Volumen (34) begrenzt, die mit den Verbindungskreisen (42) und (41) zwischen der Pumpe (11) und der Durchgangsöffnung (12) sowie zwischen der Pumpe (11) und der Kammer (15) des Reservoirs (9) in Verbindung steht.

9. Chirurgisches Implantat nach Anspruch 8, **dadurch gekennzeichnet, daß** die Wand (35) der Pumpe sich im wesentlichen über die gesamte Höhe des Zylinders (18) erstreckt und dabei zur Unterstützung ihrer Betätigung mit Rillen versehen ist.

10. Chirurgisches Implantat nach Anspruch 7 oder 8, **dadurch gekennzeichnet, daß**:
- der Verbindungskreis (40) zwischen der Kammer (15) und der Durchgangsöffnung (12) mit einer kalibrierten Austrittsöffnung (45) versehen ist, um den Druck des aufblasbaren Elements (2) zu regulieren,
- der Verbindungskreis (41) zwischen der Pumpe (11) und der Kammer (15) mit dem Einweg-Verschluß (47), der in seine geschlossene Ruhestellung elastisch zurückgestellt wird, versehen ist.

11. Chirurgisches Implantat nach Anspruch 1 oder 10, **dadurch gekennzeichnet, daß** es einen Steuerschalter (60) zum Schließen des Verbindungskreises (40) zwischen dem Reservoir (9) und dem aufblasbaren Element (2) umfaßt.

12. Chirurgisches Implantat nach Anspruch 11, **dadurch gekennzeichnet, daß** der Steuerschalter (60) auf beiden Seiten des Steuergehäuses (7) zugänglich ist.

13. Chirurgisches Implantat nach einem der Ansprüche 1 bis 12, **dadurch gekennzeichnet, daß** das aufblasbare Element (2) in Form einer Manschette (3) vorliegt, die geeignet ist, ein Organ, das mit einem Innendurchgang versehen ist, zu umschließen, damit er durch Zusammenschnüren durch das aufblasbare Element geschlossen oder geöffnet wird.
